# EUROPEAN PATENT SPECIFICATION

(11) **EP 0 427 457 B1**
(45) Date of publication and mention of the grant of the patent: **01.03.1995**
(21) Application number: 90311965.9
(22) Date of filing: 01.11.1990
(51) Int. Cl.: A61M 5/307

(54) **Ampule for Needleless Hypodermic Injection Device**
Ampulle für nadelloses, hypodermisches Injektionsgerät
Ampoule pour dispositif d'injection hypodermique sans aiguille

(30) Priority: 09.11.1989 US 434250; 02.07.1990 US 547898
(43) Date of publication of application: 15.05.1991
(73) Proprietor: BIOJECT INC, Porland, Oregon 97224 (US)
(72) Inventor: McKinnon, Charles Neal, Laguna Niguel, California 92677 (US); Nakagawa, Takaaki, Tualatin, Oregon 97062 (US); Wilcox, Carl Evan, Porland, Oregon 97224 (US)
(74) Representative: Senior, Alan Murray

(56) References cited:
- EP-A- 0 008 636
- EP-A- 0 119 286
- FR-A- 2 627 698
- GB-A- 2 193 644
- US-A- 4 790 824

## Description

The field of the present invention is needleless hypodermic injection devices. More particularly, the invention relates to needleless hypodermic devices utilizing pressurized gas for injection of medication.

Various needleless hypodermic injection devices have been known in the past. For example, Morrow et al, U.S. Patent 4,790,824 describes a needleless hypodermic injection device having a two-stage gas delivery system and an ampule shroud containing medication which is driven through the skin via gas pressure.

Parsons et al, U.S. Patent No. 4,680,027 discloses an injection device using a pressurized gas cartridge to drive a piston against the biasing force of a spring. The driven piston works on a syringe causing liquid medication to be ejected with sufficient pressure to penetrate the skin of the patient.

While these and other known injection devices have met with varying degrees of success, their constructions or operating features can prevent effective injection. It has now been discovered that the injection of liquid medication or injectant should be as instantaneous as possible. With gas powered injection devices, the rise time of the gas pressure acting on the piston, and the resulting acceleration at which the piston and injectant are driven is critical. When the gas pressure acting on the piston rises too slowly, the initial medication ejected from the device does not have sufficient pressure or velocity to pass through the skin. In addition, if the "rise time" of the injection sequence is not sufficiently fast, a substantial portion of the medication will be too slowly driven from the device causing a "splash back" condition. Consequently, as a result of "splash back" the patient does not receive the full dosage of medication.

In gas driven injection devices, there are several factors which may effect the efficiency of the device. For example, devices having a long or tortuous gas path will have slower rise times due to flow losses and gas volume compressibility effects. In addition, certain injection devices rely on direct mechanical valve operation by the user of the device to release the gas pressure during the injection sequence. Since the valve operation is done manually in these devices, the effectiveness of the injection can vary widely with the user, due to the speed, activating force and completeness of activating movement employed by different users of the device. More importantly, it has not been previously appreciated that many of these types of devices have relatively large "dead" spaces or volumes of gas trapped behind the piston when the device in the ready to fire condition. These dead volumes substantially hinder injection by slowing the rise time of the gas pressure acting on the piston since substantial time is required for relatively large volumes of gas to flow into the dead volumes to build up an adequate injection pressure.

Gas driven injection devices can also be inadvertently activated if the valve of the device is inadvertently depressed or opened by the user, or if the user should drop the device, etc. This results in wasted injected medication and driving compressed gas.

Gas driven injection devices using compressed gas cartridges can provide a limited number of injections before the cartridge must be replaced with a fresh cartridge. With each injection, some compressed gas is expended thereby decreasing the available supply of compressed gas remaining in the device. After a certain number of injections, the available compressed gas pressure within the device becomes inadequate for proper injection. Consequently, depending on the type of device and the type of injections being provided by the device, it has been necessary for the user of the device to keep track of the number of injections provided by the cartridge in the device, and to replace the cartridge after a maximum specified number of injections. If the maximum number of injections per cartridge is exceeded, the decreased and insufficient gas pressure available can lead to "splash back" as described above.

The aforementioned US-A-4 790 824 discloses the use of an ampule in accordance with the pre-characterising portion of claim 1.

It is an object of the invention to provide an improved needleless hypodermic injection device which has a novel ampule assembly.

According to the present invention, there is provided an ampule comprising an ampule body containing a generally cylindrical injectant chamber; a nozzle section attached to the ampule body and having a nozzle opening leading towards the injectant chamber; a transition section between and adjoining the injectant chamber and the nozzle section and a tubular shield attached to the ampule body, the nozzle section being concentric with said shield; characterised by a luer fitting on the nozzle section, the luer fitting being substantially within the shield, and in that the transition section has a concave section adjoining the injectant chamber, a convex section adjoining the concave section, and a conical section between the convex section and the nozzle section.

The construction of the ampule reduces pressure losses and lowers turbulence.

Preferably, a throat extends and tapers from the injectant chamber to a nozzle at one end of the ampule. Cam flanges may also be provided at the other end of the ampule for securing the ampule to the injection device. Key tabs on the ampule adjacent and perpendicular to the flanges may be provided for disengaging a locking system of the injection device.

The present invention will be described, by way of example only, in the following detailed description, taken in connection with the accompanying drawings.

In the drawings wherein similar reference characters denote similar elements throughout the several views:
Figure l is a perspective view of the present needleless injection device in the ready for injection state;
Figure 2 is a side view in part section of the injection device of Figure 1;
Figure 3 is an enlarged fragment view in part section of the valve mechanisms and gas delivery system of the device of Figure 2;
Figure 4 is an enlarged section view taken along line 4- 4 of Figure 3;
Figure 5 is a top interior view taken along line 5-5 of Figure 4;
Figure 6 is a side view of the Luer fitting provided on the ampule shown in Figures 2 and 7; and
Figure 7 is an enlarged section view fragment of an ampule which may be used with the injection device.
Figure 8 is a perspective view of a second embodiment of the present needleless injection device in the ready for injection state;
Figure 9 is an enlarged side view in part section of the injection device of Figure 8;
Figure 10 is a front elevation view taken along line 10-10 of Figure 9;
Figure 11a is an enlarged fragment view in part section of certain features of the vice of Figure 8;
Figure 11b is an enlarged fragment view in part section of certain features of the device of Figure 8 while the device is injecting;
Figure 12 is an enlarged fragment in part of section of the device of Figure 8 with a first locking system of the device unlocked by an ampule secured to the device;
Figure 13 is an enlarged section view taken along line 13-13 of Figure 9;
Figure 14a is an enlarged fragment view in part section showing the front end of the device of Figure 8 before an ampule is secured into the device.
Figure 14b is an enlarged fragment view in part section of the front end of the device of Figure 8, as taken along line 14b-14b of Figure 13 and showing an ampule installed but not secured into the device;
Figure 14c is an enlarged fragment view in part section of the front end of the device of Figure 8, with an ampule installed and secured into the device;
Figure 15 is an enlarged section view taken along line 15-15 of Figure 14c;
Figure 16 is an exploded perspective view of an ampule, a retainer and a detent collar of the device of Figure 8;
Figure 17a is a front elevation view in part section showing the ampule inserted into the retainer of Figure 16 but not secured therein;
Figure 17b is a front elevation view in part section showing the ampule rotated and secured into the retainer of Figure 16;
Figure 18 is a front elevation view of the ampule of Figure 16;
Figure 19 is a rear elevation view of the ampule of Figure 16;
Figure 20 is a section view of the ampule taken along line 20-20 of Figure 19;
Figure 21 is a front elevation view of an adapter plate to facilitate use of existing ampules with the device of Figure 8;
Figure 22 is a bottom elevation view of the adapter plate of Figure 21;
Figure 23 is a side elevation view of the adapter plate of Figure 21 on an existing ampule;
Figure 24 is an enlarged front elevation view in part section of the collar of Figure 16;
Figure 25 is a section view of the collar taken along line 25-25 of Figure 24;
Figure 26 is a section view of the a third embodiment of the present needleless injection device in the ready for injection condition;
Figure 27 is an enlarged section view of an ampule for use with the injection device of Figure 26;
Figure 28 is an enlarged section view fragment of the nozzle of the ampule of Figure 27; and
Figure 29 is an enlarged perspective view fragment of the nozzle end of the ampule of Figure 27.

### Detailed Description of the Preferred Embodiments

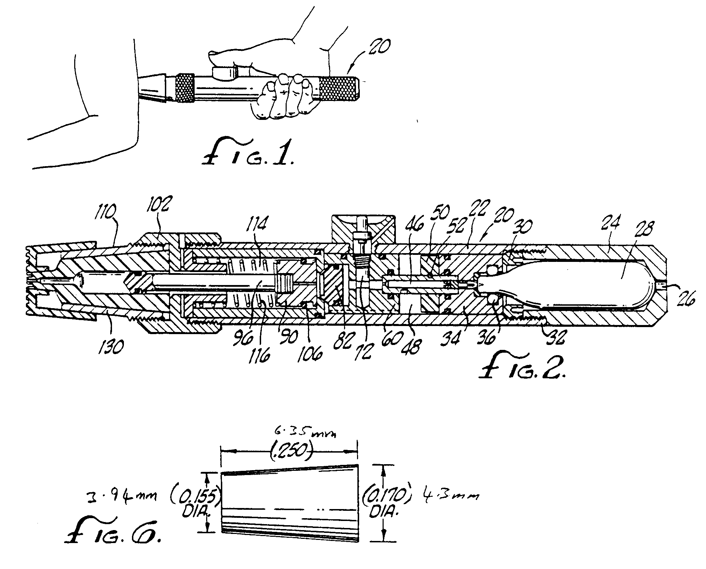

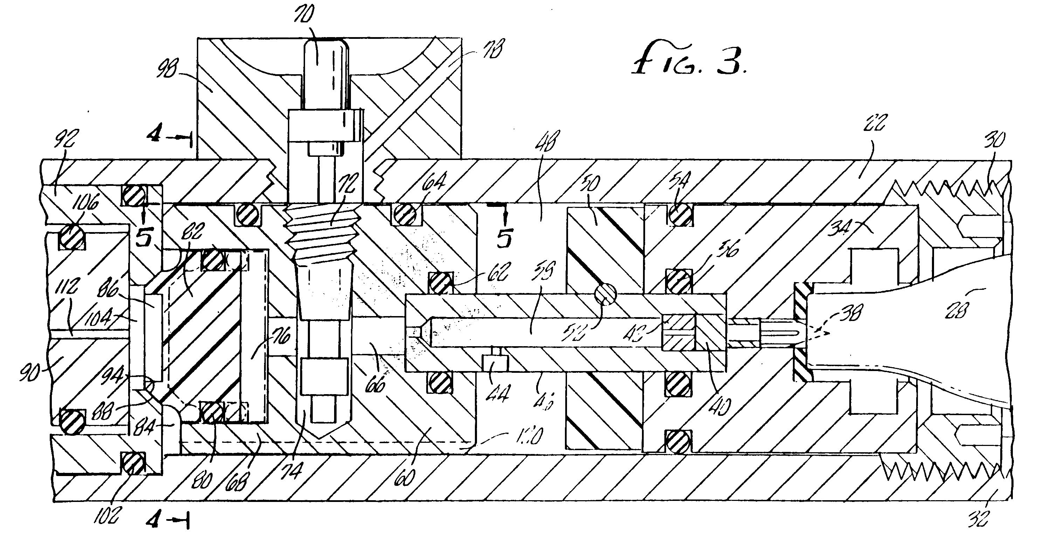

Referring now in detail to the drawings, therein illustrated is a novel needleless hypodermic injection device, which as shown in Figures 1 and 2 includes a housing 22 and a cartridge holder 24 threaded onto an internal threaded section 32 of the housing 22 and holding a cartridge 28. At room temperature, the cartridge 28 contains a saturated gas/liquid, such as CO₂ or some other appropriate pressure medium, hereinafter referred to as "compressed gas". An opening 26 is provided in the cartridge holder 24. A spanner collar 30 is threaded onto the internal threaded section 32 of the housing 22 ahead of the cartridge holder 24 and serves to hold other internal components in place.

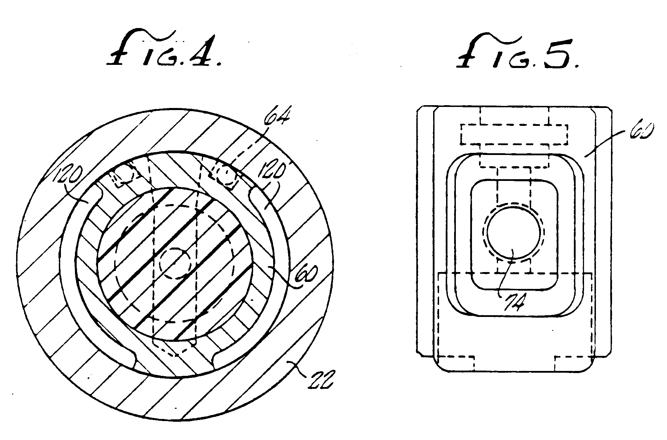

Turning to Figure 3, a piercing body 34 is contained within the housing 22 against the spanner collar 30. An elastomeric washer 36 within the piercing body seals against the facing end of the cartridge 28. A slotted piercing pin 38 extends outwardly from the piercing body 34 into the cartridge 28. The piercing pin 38 connects to a gas delivery tube 46. A filter 40 and an orifice 42 are secured within the gas delivery tube 46.

Spaced apart from the piercing body 34 within the housing 22 is a valve body 60. The valve body 60, housing 22, and piercing body 34 form a reservoir 48 around the gas delivery tube 46. A bleed hole 44 leads from the gas delivery tube to the reservoir 48. A spacer 50 is optionally secured around the gas delivery tube 46 within the reservoir 48 by a pin 52.

An O-ring 54 seals the piercing body against the inner surface of the housing 22. Similarly, an O-ring 56 seals the gas delivery tube 46 to the piercing body 34.

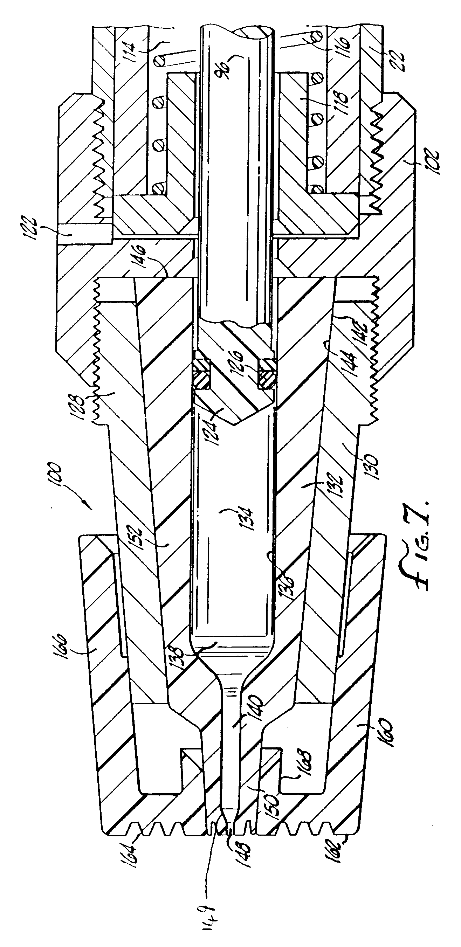

At the other end of the gas delivery tube 46, an O-ring 62 seals the gas delivery tube 46 to the valve body 60, with the gas delivery tube extending into a bore 66 running through the valve body 60. A pilot valve 72, preferably a Schrader-type poppet valve, is contained within a pilot valve chamber 74 within the valve body 60. The valve body 60 includes a sleeve section 68 substantially containing a main valve piston 82. An O-ring 80 seals the main valve piston 82 against the inner surfaces of the sleeve section 68 of the valve body 60 with the main valve piston 82 slidably disposed therein. In between the main valve piston 82 and the pilot valve chamber 74 is a main piston chamber 76, with the bore 66 also extending from the pilot valve chamber 74 to the main piston chamber 76. As shown in Figure 4, gas passageways 120 extend along the periphery of the valve body 60 to connect the reservoir 48 to the main piston chamber 76. The gas passageways 120 are designed to maximize unrestricted flow from the reservoir 48 into the plunger chamber 114.

The main valve piston 82 includes a piston face 88 for sealing against a valve or liner seat 94 of a liner 92. Alternatively, the seat may be formed on an inwardly extending annular rim section of the housing 22. The main valve piston 82 and liner seat 94 form the main valve of the injection device. The main valve piston 82 is advantageously made of TEFLON (Trademark) and also preferably has a counter bore 88 for improved sealing characteristics.

A button 70 is held against the pilot valve 72 within a button housing 98. An O-Ring 64 seals the valve body 60 to the inner surface of the housing 22, around the pilot valve 72 which passes partially through the housing 22. A vent 78 extends through the button housing 98 into the pilot valve chamber 74.

The liner 92 has an opening 104 adjacent the liner seat 94 and is sealed against the inner surface of the housing 22 by O-ring 102. The sleeve section 68, the liner 92 and the main valve piston 82 form a seat chamber or annular chamber 84 on the side of the main valve piston 82 opposite of the main piston chamber 76.

Within the liner 92 is a plunger driver 90 engaging a plunger 96. A plunger driver orifice 112 extends through the plunger driver 90 connecting the liner opening 104 to the plunger chamber 114. As shown in Figure 2, a compression spring 116 is positioned on a hub 118 extending into the plunger chamber 114. The spring 116 biases the plunger driver 90 against the end of the liner 92 adjacent the liner seat 94. An O-ring 106 seals the plunger driver 90 against the liner 92.

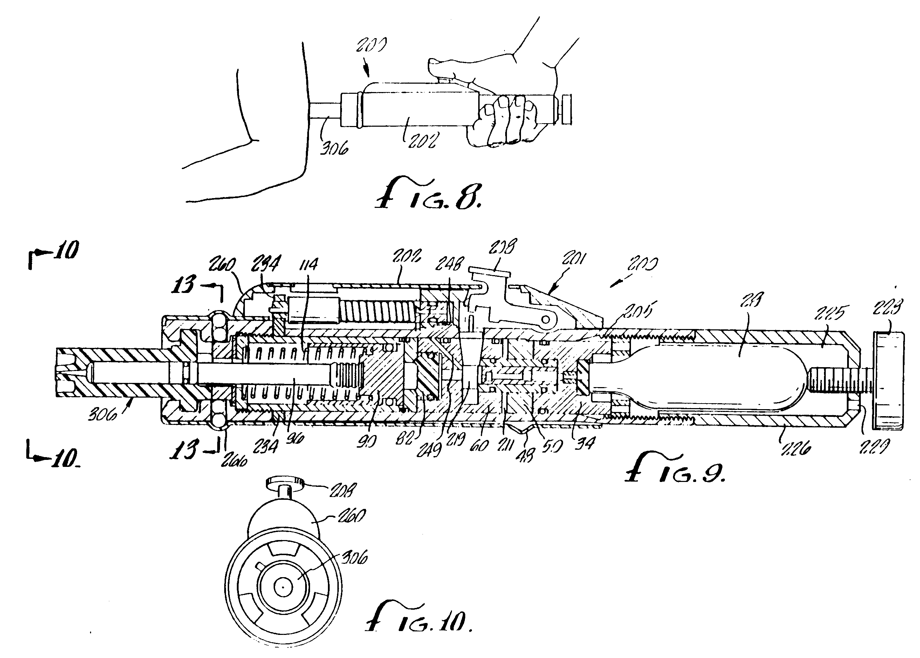

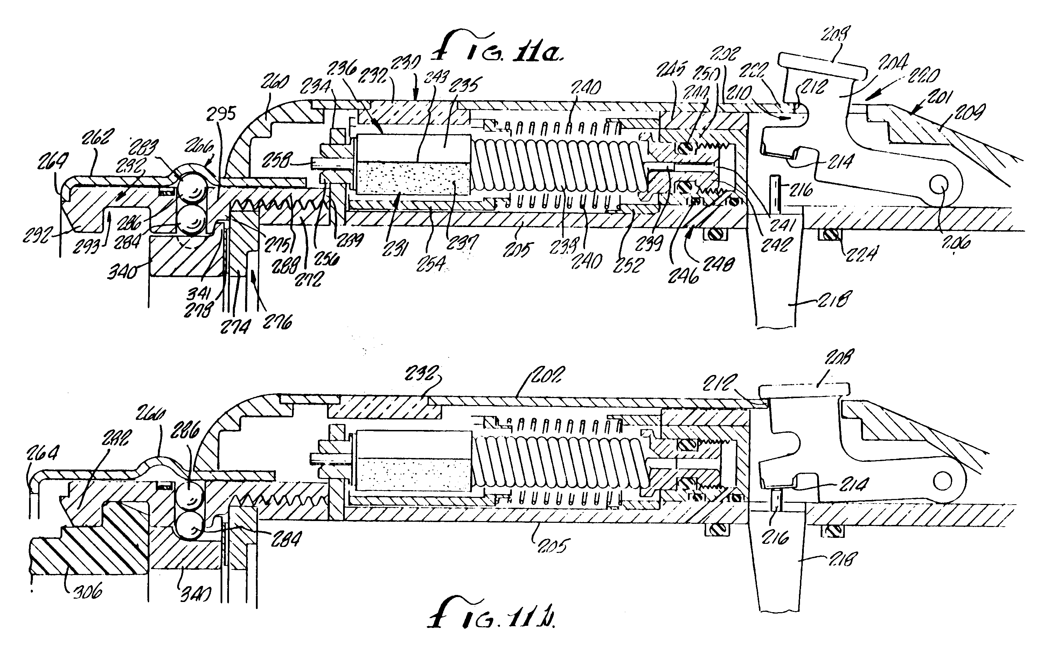

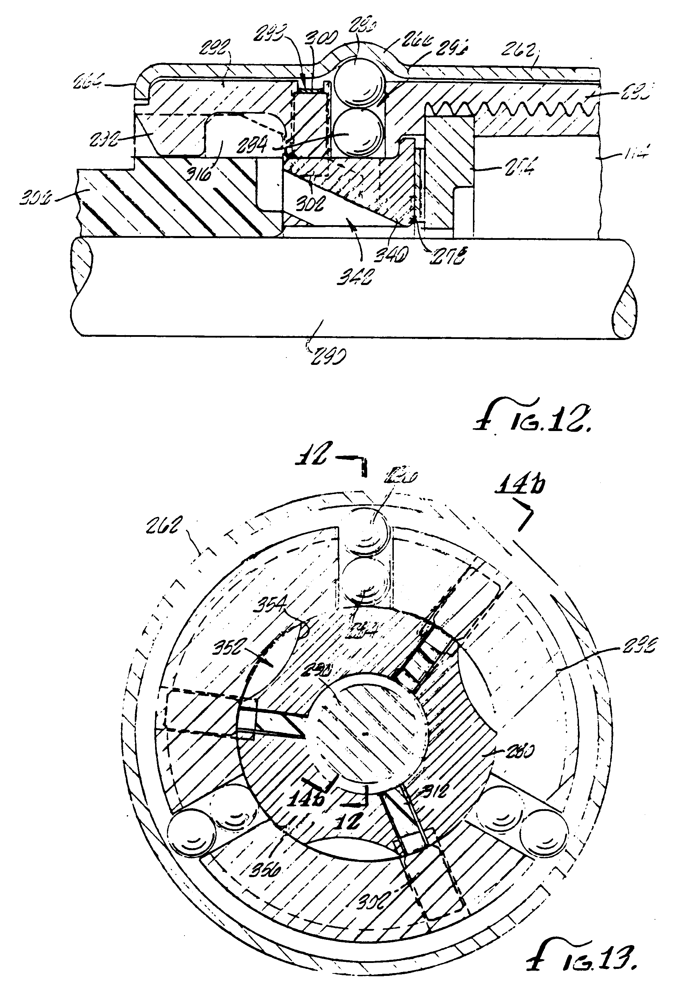

Referring now to Figure 7, a threaded collar 102 having a vent 122 leading to the plunger chamber 114 joins the housing 22 and a threaded end 128 of an ampule holder 130. Within the ampule holder 130 is an ampule 132 having a base 146 abutting the threaded collar 102. The conically tapered inner surface of the ampule holder 144 matches the conically tapered outer surface 142 of the ampule 132, to support the ampule walls 152 all around. With shallow angles of taper, there is a tendency for the ampule 132 to stick in the ampule holder 130 after the injection. This is called a locking taper. The ampule 132 and ampule holder 130 preferably have approximately a 16° taper which is advantageously above the locking taper range. The ampule 132 is advantageously a single moulded piece. A breech lock fitting may alternately be provided for quick ampule replacement.

The plunger 96, which has a tapered end 124 and, in a groove, an "O" ring seal 126 and back-up ring extends into an injectant chamber 134 within the ampule 132. The plunger seal 126 seals the plunger 124 against the injectant chamber walls 136, which are substantially parallel. The injectant chamber 134 leads to a flow path comprising a first transition 138 extending into a throat 140 leading to a second 149 and a nozzle 148. The flow path smoothly makes the cross-section area reduction from the injectant chamber 134 to the nozzle 148, to minimise flow losses. Around the outside of the nozzle end of the throat 140 of the ampule 132 is an ANSI standard Luer fitting 150 as detailed in Figure 6. This fitting permits connection of the ampule 132 to another medical device or container in a leak-proof and mechanically secure manner. Advantageously, an ampule 132 made of or lined with glass or another material which does not interact with the desired injectant may be used. The injectant chamber 134, first transition 138, throat 140, second transition 149 and nozzle 148 are preferably moulded in as part of the ampule during its manufacture.

A shield 160 is preferably provided over the ampule 132 and at least part of the ampule holder 130. The shield 160 includes a Luer sleeve 168 adapted to fit over the Luer fitting 150 of the ampule 132. The front surface 162 of the shield 160 has ridges 164. A cylindrical flange 166 extends back from the front surface 162 and engages the ampule holder 130.

In operation, the cartridge holder 24 is unscrewed from the housing 22 of the injection device 20. A compressed gas cartridge 28, such as a C0₂ cartridge is placed into the cartridge holder 24. The cartridge holder 24 is then threaded back onto the internal threaded section 32 of the housing 22. As the cartridge holder 24 is turned to engage the housing 22, the face end of the neck of the cartridge 24 engages, compresses and seals against the elastomeric washer 36 and the cartridge 28 is pierced by the piercing pin 38 projecting from the piercing body 34. After the cartridge 28 is pierced, compressed gas flows by the piercing pin 38, through the filter 40 and the orifice 42. The filter 40 traps any contaminants in the gas flow. The orifice 42 limits the flow rate.

Compressed gas flows through the 42 and fills the duct 58 within the gas delivery tube 46. The gas continues to flow and fill the bore 66, pilot valve chamber 74 and the main piston chamber 76. Simultaneously, gas flows from the duct 58 through the bleed hole 44 in the gas delivery tube 46 to fill the reservoir 48. From the reservoir 48 the gas flows through the gas passageways 120 to fill the annular chamber 84. After a sufficient interval, all chambers, spaces and flow channels are at a pressure P1.

The spacer 50 is provided in the reservoir 48 to allow the volume of gas contained in the reservoir to be varied. This capability of varying volume enables the device to be used for subcutaneous (usually relatively smaller volumes) and intramuscular (relatively larger volumes) injection. The bleed hole 44 is positioned adjacent to the valve body 60 such that a wider spacer 50 may be provided without interfering with the bleed hole 44. The diameter of the bleed hole 44 is small in comparison to the flow areas of the duct 58, bore 66 and gas passageways 120.

With device in the ready state, as described above and as illustrated in Figure 3, the piston face 88 of the main valve piston 82 is sealed against the liner seat 94 of the liner 92, such that no gas may flow through the liner opening 104. The main valve piston 82 is forced against the liner seat 94 to make the seal by virtue of the pressure exerted on the back of the main valve piston, i.e. the surface facing the main piston chamber 76. Although in the ready state, the annular chamber 84 and in the main piston chamber 76 have equal gas pressure, the projected area of the main valve piston 82 facing the main piston chamber 76 is greater than the projected area of the main valve piston 82 facing the annular chamber 84. The resulting force imbalance causes the main valve piston 82 to be tightly sealed against the liner seat 94.

Using known techniques, the desired injectant is loaded into the ampule 132. Single-use ampules may be provided as a unit along with the plunger 96 and the shield 160. Unit-does ampules prefilled with injectant may also be used. These ampules have a relatively larger surface around the nozzle 148 and no Luer fitting. Correspondingly, the throat 140 of such ampules may be shortened. The base 146 of prefilled ampules is sealed with a plug or membrane.

With the ampule 132 loaded with injectant, the ampule holder 130 is placed over the ampule 132 and the threaded end 128 of the ampule holder 130 is engaged by the threaded collar 102. The plunger 96 passes through the threaded collar 102 and extends into the plunger driver 90 in the plunger chamber 114. (See Figure 2.) The injection device is then ready for injection, by placing the device 20 against the patient's skin. (See Figure 1).

As the front surface of the ampule 132 is relatively small, the shield 160 advantageously is provided over the Luer fitting 150 of the ampule 132 to help steady the injection device 20 against the patient's skin. The ridges 164 on the front surface 162 of the shield 160 help to prevent sliding over the patient's skin and local anesthetic phenomenon. The flange 166 of the shield 160 covers the ampule holder surface 30 and is intended to help to prevent bodily fluids from contacting the reusable ampule holder 130.

During the injection sequence, substantial pressure is developed within the injectant chamber 134. Consequently, it is advantageous to avoid overstressing the injectant chamber walls 136. The ampule holder 130 may help to prevent excessively stressing the Luer fitting 150, the transition 138 or the injectant chamber walls 136 of the ampule 132 by at least partially transferring stresses (which may be generated by lateral or bending movement of the nozzle 148 against the patient's skin) to the ampule holder 130.

With the device 20 in the ready state and held against the patient's skin, the device 20 is activated by depressing the button 70. This causes the pilot valve 72 to open permitting the compressed gas in the pilot valve chamber 74 to escape through vent 78 to the outside. Simultaneously, the gas in the main piston chamber 76 rushes outwardly along the same path causing a substantial pressure drop therein. The small diameter of the bleed hole 44 in the gas delivery tube 46 severely restricts the flow of gas from the annular chamber 84 through the gas passageways 120 and reservoir 48 into the duct 58. Similarly, the orifice 42 severely restricts the flow of gas from the cartridge 28. As a result, at the instant just after the button 70 is depressed, the pressure in the annular chamber 84 is far higher than the pressure in the main piston chamber 76. The main valve piston 82 is thereby rapidly driven in a snap action backwards towards the pilot valve chamber 74 such that the seal between the piston face 88 and the liner 94 is opened, as shown in phantom in Figure 3. The gas in the reservoir 48 is then able to flow through the gas passageway 120 and through the opening 104 into the plunger chamber 114 to drive the plunger driver 90 and plunger 96 into the injectant chamber 134 of the ampule 132. As the plunger 96 and plunger driver 90 move outwardly toward the ampule 130, the plunger chamber 114 is vented through the plunger chamber vent 122. The rapid acceleration of the plunger 96 causes the injectant to be injected out of the nozzle 148 at a pressure and velocity sufficient to pass through the patient's skin.

During the injection sequence, a small amount of compressed "bleed" gas may also flow from the cartridge 28 and reservoir 48 into the pilot valve chamber and out through the vent 78. However, this quantity of gas is acceptably small in comparison to the "driving" gas flowing from the reservoir 48 into the plunger chamber 114. In addition, since the volume of the reservoir 48 is large compared to the initial "dead" volume between plunger driver 90 and the main valve, the rise in gas pressure acting on the plunger driver 90 is very fast.

Following the injection, the button 70 is released and the pilot valve 72 closes. The gas pressures in the various ducts and chambers within the housing 22 then once again equalize and return substantially to Pl. Specifically, compressed gas from the cartridge 28 flows through the bleed hole 44 to repressurize the reservoir 48 and through the duct 58 to repressurize the bore 66 pilot valve chamber 74 and main piston chamber 76. Due to the small size of the openings in the orifice 42 and the bleed hole 44, this repressurization occurs slowly in comparison to the injection sequence. As the main piston chamber 76 is repressurized, the main valve piston 82 is driven forward so that the piston face 88 once again seals against the liner seat 94. In the plunger chamber 114, the spring 116 pushes the plunger driver 90 back against the opening 104. As the plunger driver 90 is returned to its original ready position, remaining gas in the plunger chamber 114 vents through the plunger driver orifice 112.

To prepare the device 20 for the next injection, the ampule holder 130, the ampule 132 and the plunger 96 are removed from the housing 22 by unscrewing the ampule holder 130 from the threaded collar 102. A new ampule assembly 100 consisting of a new filled ampule 132, plunger 96, and shield 160 are installed on the device 20 as previously described.

Depending upon the particular application, the gas cartridge 28 is sufficient for several injections. To replace the cartridge 28 after a predetermined number of injections, the cartridge holder 24 is unscrewed from the housing 22. As the cartridge holder 24 is being unscrewed from the housing 22, remaining compressed gas in the cartridge 28 may escape from the cartridge 28 into the interior of the cartridge holder 24. The opening 26 at the end of the cartridge holder 24 prevents the cartridge holder 24 from becoming pressurized, such that the cartridge holder 24 may be easily removed from the housing 22. A new gas cartridge 28 is then installed as previously described.

The device 20 may be used for intramuscular or subcutaneous injections. For subcutaneous injection, the nozzle 148 has a relatively smaller opening and the reservoir 48 is largely occupied by a spacer 50, limiting the gas volume therein to preferably as little as 20% of the full reservoir volume. For intramuscular injection, a larger nozzle 148 opening advantageously used and the reservoir may be up to 100% filled with "driving" gas, i.e., no spacer is used. The nozzle 148 opening may range in diameter from approximately .004 to .025 inches. The nozzle diameter and spacer 50 size (or length) determine whether the device is set up for intramuscular or subcutaneous injection.

Various other design alternatives will be apparent to those skilled in the art. For example, the various O-rings which seal non-moving components within the housing 22 may be replaced or eliminated by other types of seals (including adhesives) or internal construction. In addition, a diaphragm or bellows could be used in place of the main valve piston 82 and various other configurations of the valves, chambers and flow passageways are also possible.

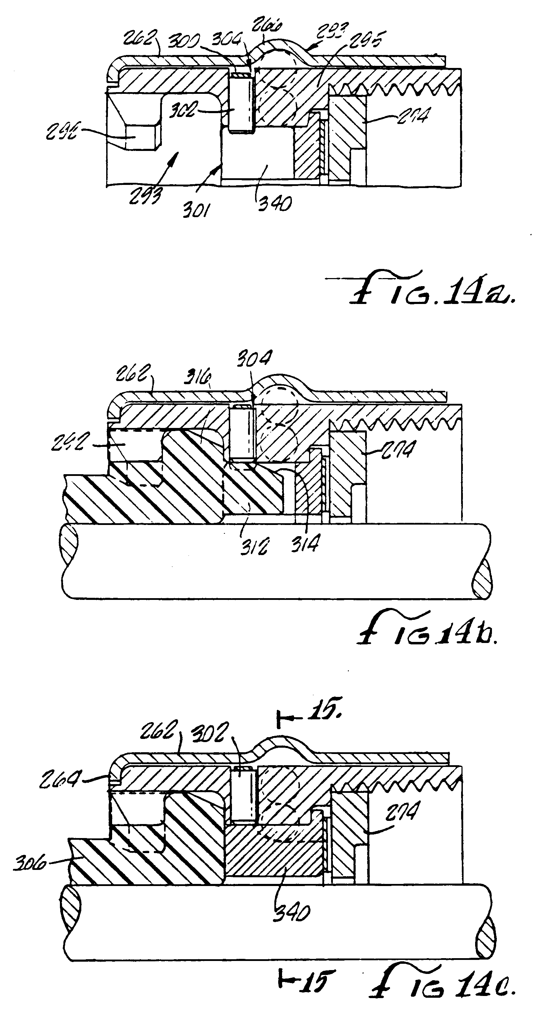

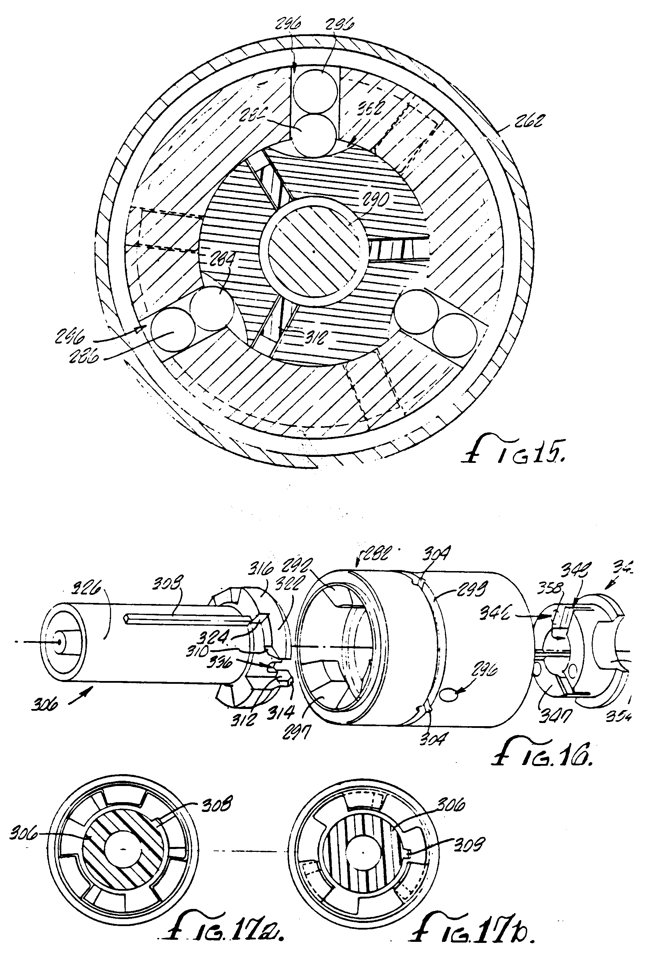

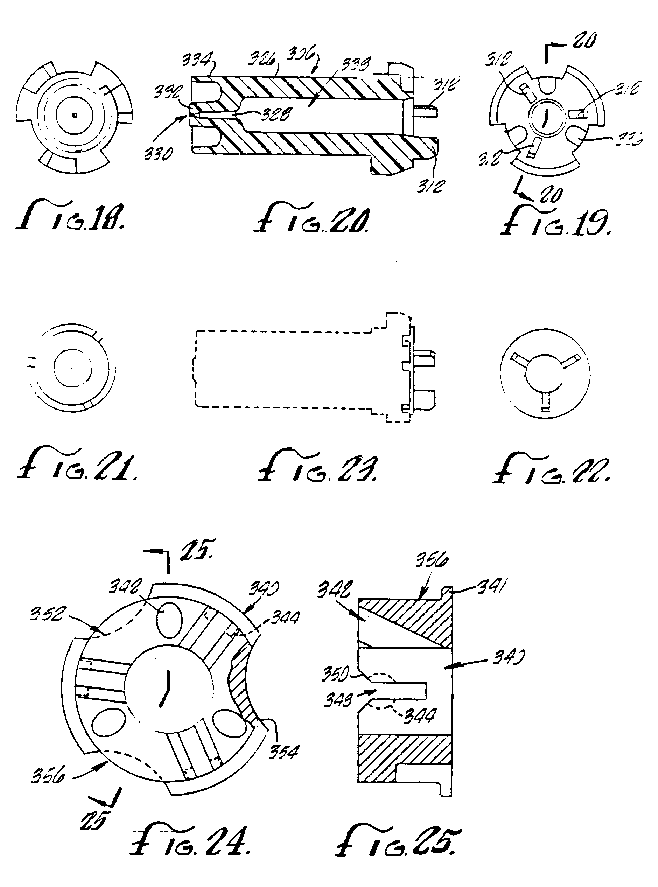

Figures 8-25 illustrate a second embodiment of the injection device and an ampule for use with this device. The second embodiment includes a compressed gas pressure indicator and interlock systems.

Referring to Figures 8, 9 and 10, the second embodiment of the device 200 has a tubular housing 205 containing substantially the same elements as shown in Figure 3. The structure and operation of these elements within the housing 205 are substantially the same as those described above and illustrated in Figure 2-5. A gas cartridge holder 226 holding a cartridge 28 is threaded on to the back or cartridge end of the housing 205. A thumb screw 228 is threaded through the cartridge holder 226 and extends into the cartridge chamber 225. A vent 229 extends through the cartridge holder 226.

Referring specifically to Figure 9, a slide assembly 201 having a slide tube 202 is fitted over the housing 205. A trigger 204 is pivotally mounted onto the housing 205 by a trigger pin 206 and protrudes through the top of the slide assembly 201.

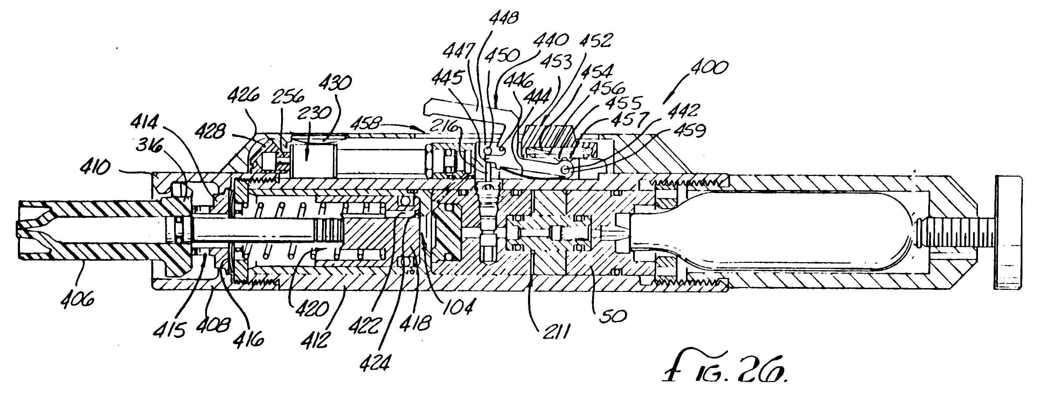

Referring now to Figure 11a, the slide assembly 201 has a ramp 209 extending at a incline up to a trigger opening 220 in the slide tube 202. A thumb rest 208 of the trigger 204 extends through the trigger opening 220. A planer trigger anvil 214 on the trigger 204 is positioned over a pilot valve pin 216 for actuating a pilot valve 218. The trigger 204 has a trigger slot 210. A slide overhang 222 of the slide tube 202 has a trigger stop 212 extending into the trigger slot 210. The trigger stop 212 prevents the trigger 204 from depressing the pilot valve pin 216 to actuate the pilot valve 218, except when the device is unlocked during an injection.

Forward of the trigger 204 is a compressed gas pressure indicator assembly 230. The indicator assembly includes a Bourdon tube 238 having an open end extending into an adaptor 242 threaded into an end cap 250 which is sealed against a flattened portion of the housing 205 with an 0-ring 246. The other side (top) of the end cap 250 is spaced apart from the slide tube 202 by a slide bushing 245. An 0-ring 244 seals the adaptor 242 against the end cap 250. Similarly, an 0-ring 224 seals the pilot valve 218 against the inside surface of the housing 205 A spring cup 252 surrounding the adapter 242 supports the back end of a compression spring 240. The compression spring 240 extends forward over the Bourdon tube 238 to a barrel 254 substantially surrounding an indicator cylinder 231. The indicator cylinder 231 is provided with a flat 236 having two different colored sections 235 and 237, for example red and green. The two colored sections 235 and 237 are separated by a demarcation line 243.

A shaft 258 extends from the indicator cylinder 231 through a rotation bushing 256. An end support 234 mounted to the housing 205 supports the rotation bushing 256. A window 232 made of a transparent material is provided in the slide tube 202 over the indicator cylinder 231. The window 232 is preferably provided with an axially extending reference line.

Referring to Figures 9 and 11a, the open end 239 of the Bourdon tube 238 connects to an opening 241 extending through the adaptor 242 and to a duct 248 passing through the bottom wall of the adaptor 242 and through the housing 205. As shown in Figure 9, a duct extension 249 connecting to the duct 248 passes through the valve body 60 to the pilot valve chamber 219.

Referring to Figure 11a, a bridge radius 260 extends from the slide tube 202 overlying the indicator assembly 230 and is joined to a forward extension 262 of the slide tube 202. As shown in Figures 9 and 11a, the forward extension 262 of the slide tube 202 has a lock groove 266 forward of the connection to the bridge radius 260. An inwardly extending rim 264 is provided at the other end of the forward extension 262.

A retainer 282 within the slide tube 202 has three equally radially spaced apart hooks 292 (see Figure 16). A threaded end of the retainer is screwed on to a threaded end 272 of the housing 272, with the retainer shoulder 289 clamping the end support 234 against the housing 205. A plunger chamber end cap 274 is secured in between a retainer boss 275 and the threaded end of the housing 272. A detent collar 340 is held in place within the retainer 282 with a detent collar ring 341 slidably held against the retainer boss 275 by a wavy spring 278.

As illustrated in Figure 16, 24 and 25, the detent collar 340 has a cylindrical collar surface 356 interrupted by three equally radially spaced apart crescents 352 extending forward from the detent collar ring 341 to approximately the midpoint of the length of the detent collar 340. In between the three crescent surfaces 354 are three equally radially spaced apart slots 348 extending from the front surface 347 of the detent collar 340 rearward the detent collar ring 341. Chamfered guides 350 are provided at the front surface of the detent collar 347 on either side of each slot 348. A collar plunger bore 358 extends axially and centrally through the detent collar 340. As shown in Figures 24 and 25, adjacent to each slot guide 350 is a link or slide pin socket 344. The sockets 344 extend just slightly into the collar surface 356. Pressure relief bores 342 extend through the detent collar 340 from the front surface 347 into the collar plunger bore 358.

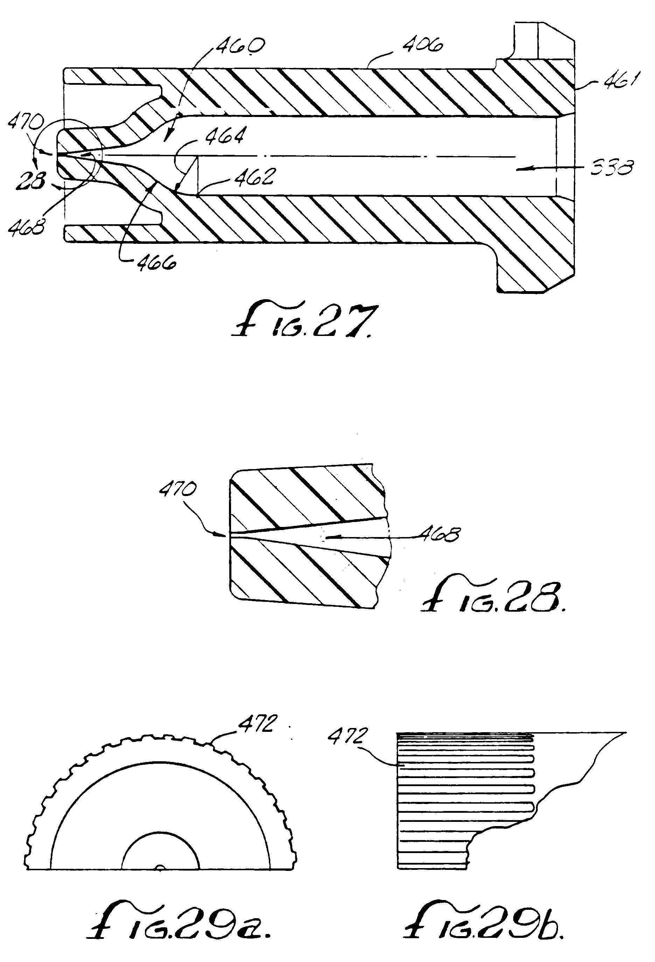

The retainer 282, as shown in Figure 11a and 14a-c has an ampule slot 293 in between the retainer hooks 292 and the retainer body 295. (In Figs. 14a-c the bridge radius and housing are omitted for clarity.) The detent collar 340 is positioned inside of the retainer body 295. As shown in Figure 14a, slide pin holes 304 extend through the retainer body 295. Slide pins 203 within the holes 304 are radially biased inwardly onto the collar surface 356 of the detent collar 340 by a clock spring 300 overlying the slide pins 302 in a clock spring groove 298. The slide pins 302 are adapted to protrude into the link or slide pin sockets 344 on the detent collar 340, to reversibly lock the detent collar from rotation within the retainer 282. Various alternative collar-retainer link configurations to link or lock the detent collar from rotating in the retainer are feasible.

In the retainer body 295 behind the slide pin holes 304 is a detent collar follower indicated generally as 283. In the preferred embodiment, the detent collar follower 283 includes inner and outer balls 284 and 286 within three detent ball bores 296. The lower balls 284 rest on the collar surface 356 such that the outside balls 286 protrude into the lock groove 266 of the forward extension 262 of the slide tube 202. Other detent collar follower configurations including pins and expandable rings are also possible.

In use, a compressed gas cartridge 28 is first installed into the device 200. Specifically, the cartridge holder 226 is unscrewed from the housing 205 and a cartridge 28 is inserted into the cartridge chamber 28 with the thumbscrew 228 substantially backed out of the cartridge chamber 225. The cartridge holder 266 is then threaded back onto the housing 205 with the neck of the cartridge 228 facing the piercing pin 38 and the elastomeric washer 36 (see Figs. 3 and 9). The thumbscrew 228 is then turned inwardly or forward to force the compressed gas cartridge 28 onto the piercing pin 38. Compressed gas then flows from the compressed gas cartridge 28 into the device as described above for the device of Figs. 1-3.

As shown in Figs. 11a and 14a, no ampule 306 is yet installed in the device 200 and the device 200 is in a locked condition. The trigger 204 cannot be depressed to actuate the pilot valve 218 because the trigger stop 212 holds the trigger anvil 214 away from the pilot valve pin 216. The trigger stop 212 cannot be pushed or slid forward out of the trigger slot 210 because the lock groove 266 on the forward extension 262 of the slide tube 202 interferes with and cannot pass over the outer ball 286 of the collar follower 283. The outer balls 286 are held in the "up" position into the lock groove 266 by the lower balls 284 which in turn are resting on the collar surfaces 356 of the detent collar 340. The detent collar 340 is held in position relative to the retainer 282 slide pins 302 biased into the slide pin sockets 344 on the detent collar 340, by the clock spring 300. Consequently, in this state, the crescents 352 on the detent collar 340 cannot be moved into alignment with the inner balls 284. Thus with no ampule secure into the device 200, the device cannot be actuated and no compressed gas can be released.

Referring now to Figures 12, 13, 14b and 20, an ampule 306 containing an injectant in the injectant chamber 388 is inserted into the front end of the device 200 by pushing in and turning within the retainer 282. The radial or key tabs 312 on the back of the ampule 306 are aligned with the slots 348 in the detent collar 340. The flanges 316 of the ampule 306 pass in between the retainer hooks 292 as the ampule 306 is inserted.

As the radial tabs 312 slide into the slots 348, the slide pins 302 ride up on the outside surfaces of the radial tabs 312 causing the slide pins 302 to lift out of the slide pin sockets 344. The detent collar 340 is then free to rotate within the retainer 282. This condition is shown in Figures 13 and 14b wherein the slide pins 302 (shown in phantom) are pushed out of the slide pin sockets 344 by the radial tabs 312 against the force of the clock spring 300. Although this link or slide pin locking system for locking the detent collar 280 to the retainer 282 is unlocked or disengaged, the device cannot be activated as the outer balls 286 still prevent forward movement of the slide tube 202 thereby preventing removal of the trigger stop 212 from the trigger slot 210.

The ampule 306 is then rotated along with the detent collar 340 within the retainer 282. The grip tab 308 on the ampule 306 provides a finger grip for gripping the ampule. While the ampule is turned it is also slightly pressed towards the back of the device 200 thereby compressing the wavy spring 278. The cams 310 on the cam arms 322 of the flange 316 on the back of the ampule 306 slide and wedge under the retainer hooks 292 (see Fig. 16).

The ampule 306 is turned until the stops 324 come to rest against the sides 297 of the retainer hooks 292, as shown in Figs. 17a and 17b. As the detent collar 340 rotates with the ampule 306, the crescents 354 come into alignment with the detent ball bores 296, thereby allowing the inner balls 284 to come to rest on the crescent surfaces 354. This allows the outer balls 286 to drop from the lock groove 266 as illustrated in Figs. 14c and 15.

The link interlock 301 (comprising the biased slide pins 302 and sockets 344 for locking the detent collar 340 against rotation) and the collar follower interlock 283 (comprising the crescents 354 and balls 284 and 286 for preventing movement of the slide tube 202) have now both been unlocked or disabled. However, the slide tube 202 remains biased to the back of the device 200 by the compression spring 240. Consequently, the trigger stop 21 remains in the trigger slot 210 to prevent depression of the trigger 208.

To administer an injection, the device 200 is positioned with the nozzle and shroud 334 of the ampule 330 against the injection site on the patient. The user then slides the slide tube 202 forward with thumb or hand pressure on the ramp 209, overcoming the biasing force of the spring 240. This movement of the slide tube 202 and trigger 208 may be performed with one or two hands. As the slide tube 202 moves forward, the trigger stop 212 moves out of the trigger slot 210 allowing the trigger 208 to be fully depressed to actuate the injection sequence.

Fig. 11b illustrates the condition of the device 200 while injecting. The trigger stop 212 is temporarily displaced forward allowing the trigger 208 to pivot downwardly with the trigger anvil 214 depressing the pilot valve pin 216 to open the pilot valve 218. The elements within the housing 205 then operate as described above for the embodiment of Figs. 1-3. The pressure relief bores 342 extending through the collar 340 and linking with the channels 336 in the ampule 306 provide a route to the outside of the device for relief of compressed gas exhausting from the plunger chamber 114 during actuation.

After the injection, the trigger 208 is released and biased to pivot away from the pilot valve pin 216. When the slide tube 202 is released by the user, the trigger stop 212 returns into the trigger slot 210. (Fig. 11a). The lock groove 266 moves back over the detent ball bores 296. The ampule 306 is rotated in the reverse direction along with the detent collar 340 and the ampule is removed from the device 200, thereby resetting or relocking the link and collar follower locking systems. The elements within the housing 205 correspondingly return to their original positions as described above with reference to Figs. 1-3.

The indicator assembly 230 indicates whether the pressure of the compressed gas within the device 200 is sufficient for another injection. The Bourdon tube 239 is calibrated in a known manner to rotate with changes of pressure within the Bourdon tube 239. As shown in Fig. 9, the duct 248 and duct extension 249 connect the Bourdon tube 239 with the pilot valve chamber 219, such that the pressure in the Bourdon tube equals the pressure in the pilot valve chamber. As the pressure of the compressed gas in the device 200 decreases with the each injection, the pressure within the Bourdon tube correspondingly decreases. This causes the Bourdon tube 239 to turn the indicator cylinder. By looking through the window 232, the user the can view the flag 236 to determine if there is sufficient gas pressure for another injection. The Bourdon tube 238 and indicator cylinder 231 are arranged so that when the gas pressure within the device 200 is sufficiently high, the green side 237 of the flag 236 appears in the window 232. As the gas pressure decreases, the indicator cylinder 231 turns with the red side 235 of the flag 236 gradually appearing in the window 230. When the red side 235 occupies a predetermined position in the window 232, the device no longer has sufficient pressure for another injection and the cartridge 28 must be replaced.

The adapter plate of Figures 21-23 is used to operate the device 200 with other ampules not having the key tabs 312.

Figure 26 illustrates a third embodiment of the present needleless injection device. As illustrated therein, an ampule 406 is secured to a retainer barrel 408 of a housing tube 412 of the injection device 400. Retainer hooks 410 on the retainer barrel 408 engage the flanges 316 of the ampule 406. A pressure plate 414 is biased by a wavy spring 416 against the back of the ampule 406 to secure it in place within the retainer barrel 408.

A plunger driver 418, similar to plunger driver 90, has a spring bore 420 and an eccentric vent 422 leading to an orifice insert 242 leading to the liner opening 104. The offset position of the eccentric vent 422 helps insure free return of the plunger driver 418 after injection.

Adjacent to the valve body 60, the spacer 50 leaves a very small volume reservoir 211, as the remaining compressed gas filling the spaces in the various chambers and passageways (which are the same as in the previous embodiment) provide adequate volume of compressed gas for injection.

An upper housing 426 is fixed in position on the outside of the housing tube 412, e.g., with screw fasteners and/or adhesives. An anchor bushing 428 is attached to the outside of the housing tube 412 and is further secured in place by the upper housing 426. The anchor bushing 428 supports the rotation bushing 256. An enlarging window 430 is provided on the upper housing 426 to permit viewing of the compressed gas pressure indicator assembly 230.

A trigger lever 440 is pivotally mounted to the housing tube 412 by a pivot pin 442. The trigger lever 440 has a locking slot 446 and an extension 447 extending through a cut out 458 in the upper housing 426. A leaf spring 444 biases the trigger lever 440 away from the housing tube 412. A boss 445 on the trigger lever 440 rests on top of the pilot valve stem 216. A slide block 448 is slidably mounted onto the housing tube 416. A slide block pin 450 extends from the slide block 448 and is engageable into the locking slot 446 of the trigger lever 440.

A detent switch assembly 452 is provided on the slide block 448. The detent switch assembly includes a detent button 453 extending through the cut out 458, a spring 444 and a detent plate 456 having protrusions 455 and 457 which are held against a detent surface 459.

Referring to Figure 27, the ampule 406 is similar to ampule 306 (Figure 9) but has a generally flat rear surface 461 without tabs 312. These tabs are not required as the injection device of Figure 26 does not have the ampule interlock system shown in the device of Figure 9.

Referring once again to Figure 27, the ampule, which is preferably injection molded as a single part of clear LEXAN HP2 (Trademark), has an injectant chamber 338, a transition zone 460 and a nozzle 470. A first or concave radius 464 begins at a tangent point 462. A second or convex radius 466 adjoins the first radius 464. A conical taper 468 extends from the nozzle 470 to the second radius 466. This provides a smooth transition zone 460 between injectant chamber and nozzle to avoid stress concentrations in the ampule 406 and provide good injection characteristics.

As shown in Figure 28, the nozzle 470 extends a short distance before joining the conical taper 468. Preferably, the length of the nozzle 470 is approximately 0.5 mm (0.020") with a nozzle diameter which can vary, depending on application, e.g., intramuscular, subcutaneous, veterinarian, etc. Figure 18 substantially illustrates the mounting tabs 316 of the ampule 406.

As shown in Figure 29, the front portion of the shroud 334 of the nozzle 406 includes serrations 472 (instead of the finger block 308, as shown in Figure 16) to provide a gripping surface.

In use, the ampule 406 is filled with injectant and is placed into the injection device 400 by passing the flanges 316 of the ampule 406 by the retainer hooks 410 on the retainer barrel 408. The pressure plate 414 is depressed slightly against the wavy spring 416. The ampule 406 is then rotated such that the flanges 316 engage the retainer hooks 410. The pressure plate 414 then clamps the ampule 410 in place within the retainer barrel 408.

The nozzle 470 of the ampule 406 is placed onto the injection site. The user pushes the detent button 453 forward, to switch the detent switch assembly from a locked to an unlocked position. As the detent button 453 is pushed forward, the slide block 448 slides forward and the slide block pin 450 disengages from the locking slot 446 on the trigger lever 440. The device 400 is then ready to inject. The trigger lever 440 is depressed to commence the injection sequence as previously described in the other embodiments. As the plunger 418 moves forward during the injection sequence, ambient gas in the plunger driver chamber is expelled by passing through relief holes 415 in the pressure plate 414, in between the flanges 316 of the ampule 406 and out of the front of the device 400.

After the injection, the spring 444 pushes the trigger lever 440 up, once it is released. The detent button 453 is returned to the locked position and the slide block pin 450 once again engages the locking slot 456 to prevent actuation of the device 400.

## Claims

1. An ampule comprising an ampule body (132) containing a generally cylindrical injectant chamber (134); a nozzle section (148) attached to the ampule body (132) and having a nozzle opening leading towards the injectant chamber (134); a transition section (138) between and adjoining the injectant chamber (134) and the nozzle section (148) and a tubular shield (160) attached to the ampule body (132), the nozzle section (148) being concentric with said shield (160); characterised by a luer fitting (150) on the nozzle section (148), the luer fitting (150) being substantially within the shield (160), and in that the transition section (138) has a concave section adjoining the injectant chamber (134), a convex section adjoining the concave section, and a conical section between the convex section and the nozzle section (148).

2. The ampule of claim 1, wherein the conical section adjoining the nozzle section (148) comprises a throat section (140) having inner throat walls leading directly from, and tapering conically outwardly from, the nozzle opening; and the transition section (138) has as the convex section forward inner transition walls curving uniformly on a radius convexly from the throat section (140) directly into rear inner transition walls, the rear inner transition walls curving as said concave section on a radius concavely from the forward inner transition walls to the ampule body, the nozzle (148), throat (140), and transition (138) sections being substantially non-deformable and having sufficient strength to withstand injection pressures without an external support.

3. The ampule of claim 2, wherein the cylindrical injectant chamber (134) has a diameter D and the radius of the rear inner transition walls is substantially D/2.

4. The ampule of any previous claim, wherein the cylindrical injectant chamber (134) is concentric with the nozzle opening (148).

5. The ampule of any previous claim, further comprising a plurality of lug sections (312) radially projecting from the ampule body (132) and spaced apart from the nozzle section (148).

6. The ampule of any previous claim, further comprising a plunger (96) slidably engageable into the ampule body (132), the plunger (96) having an O-ring (126) and a back-up ring positioned in a groove on the plunger.

## Patentansprüche

1. Ampulle mit einem Ampullenkörper (132), mit einer im allgemeinen zylinderförmigen Injektionsmittelkammer (134), einem Düsenabschnitt (148), der an dem Ampullenkörper (132) angebracht ist und eine Düsenöffnung aufweist, die in Richtung auf die Injektionsmittelkammer (132) führt, einem Übergangsbereich (138) zwischen und angrenzend an der Injektionsmittelkammer (134) und dem Düsenabschnitt (148), und einem rohrförmigen Schild (160), das an dem Ampullenkörper (132) angebracht ist, wobei der Düsenabschnitt (148) konzentrisch zu diesem Schild (160) ist, gekennzeichnet durch ein Luer-Anschlußstück (150) auf dem Düsenabschnitt (148), wobei sich das Luer-Anschlußstück (150) im wesentlichen in dem Schild (160) befindet, und dadurch, daß der Übergangsabschnitt (138) einen konkaven Abschnitt, der an der Injektionsmittelkammer (134) angrenzt, einen konvexen Abschnitt, der an dem konkaven Abschnitt angrenzt, und einen konischen Abschnitt zwischen dem konvexen Abschnitt und dem Düsenabschnitt (148) aufweist.

2. Ampulle nach Anspruch 1, bei der der konische Abschnitt, der an dem Düsenabschnitt (148) angrenzt, einen Durchlaßabschnitt (140) umfaßt, der innere Durchlaßwände aufweist, die direkt von der Düsenöffnung weg führen und sich von dort aus konisch nach außen verjüngen, und der Übergangsabschnitt (138) als den konvexen Abschnitt vordere innere Übergangswände aufweist, die sich einheitlich auf einem Radius konvex weg von dem Durchlaßabschnitt (140) direkt in hintere innere Übergangswände wölben, wobei sich die hinteren inneren Übergangswände als der konkave Abschnitt auf einem Radius konkav weg von den vorderen inneren Übergangswänden zu dem Ampullenkörper, der Düse (148), dem Durchlaß (140) wölben und die Übergangsabschnitte (138) im wesentlichen nicht deformierbar sind und eine ausreichende Festigkeit aufweisen, um den Injektionsdrücken ohne eine Unterstützung von außen standzuhalten.

3. Ampulle nach Anspruch 2, bei der die zylinderförmige Injektionsmittelkammer (134) einen Durchmesser D aufweist und der Radius der hinteren inneren Übergangswände im wesentlichen D/2 ist.

4. Ampulle nach einem der vorhergehenden Ansprüche, bei der die zylinderförmige Injektionsmittelkammer (134) konzentrisch zu der Düsenöffnung (148) ist.

5. Ampulle nach einem der vorhergehenden Ansprüche, desweiteren mit einer Vielzahl von Ansatzabschnitten (312), die radial von dem Ampullenkörper (132) vorstehen und von dem Düsenabschnitt (148) beabstandet sind.

6. Ampulle nach einem der vorhergehenden Abschnitte, desweiteren mit einem Kolben (96), der gleitend in den Ampullenkörper (132) eingreifen kann, wobei der Kolben (96) eine O-Ring-Dichtung (126) und einen Stützring aufweist, die in einer Nut des Kolbens positioniert sind.

## Revendications

1. Ampoule comprenant un corps d'ampoule (132) contenant une chambre à injectant (134) généralement cylindrique, une section de buse (148) fixée au cors d'ampoule (132) et comportant une ouverture de buse dirigée vers la chambre à injectant (134); une section de transition (138) entre et voisine de la chambre à injectant (134) et la section de buse (148), et un élément de protection tubulaire (160) fixé au corps d'ampoule (132), la section de buse (148) étant concentrique audit élément de protection (160), caractérisée par un raccord Luer (150) sur la section de buse (148), le raccord Luer (150) étant disposé sensiblement à l'intérieur de l'élément de protection (160), et en ce que la section de transition (138) comporte une section concave contiguë à la chambre à injectant (134), une section convexe contiguë à la section concave, et une section conique entre la section convexe et la section de buse (148).

2. Ampoule selon la revendication 1, dans laquelle la section conique qui est contiguë de la section de buse (148) comprend une section d'étranglement (140) comportant des parois d'étranglement internes partant directement de l'ouverture de buse et allant en s'élargissant coniquement à partir de celle-ci, et la section de transition (138) comprend en tant que section convexe des parois de transition internes avant s'incurvant de façon uniforme selon un rayon de manière convexe à partir de la section d'étranglement (140) et se raccordant directement aux parois de transition internes arrière, les parois de transition internes arrière s'incurvant au niveau de ladite section concave selon un rayon de manière concave à partir des parois de transition interne avant vers le corps d'ampoule, la buse (148), les sections d'étranglement (140) et de transition (138) étant sensiblement non déformables et présentant une résistance suffisante pour résister aux pressions d'injection sans un support externe.

3. Ampoule selon la revendication 2, dans laquelle la chambre à injectant cylindrique (134) présente un diamètre D et le rayon des parois de transition internes arrière est sensiblement de D/2.

4. Ampoule selon l'une quelconque des revendications précédentes, dans laquelle la chambre à injectant cylindrique (134) est concentrique à l'ouverture de buse (148).

5. Ampoule selon l'une quelconque des revendications précédentes, comprenant en outre une pluralité de sections de pattes (312) faisant saillie radialement du corps d'ampoule (132) et espacées de la section de buse (148).

6. Ampoule selon l'une quelconque des revendications précédentes, comprenant en outre un plongeur (96) en engagement coulissant dans le corps d'ampoule (132), le plongeur (96) comportant un joint torique (126) et une bague d'appui positionnés dans une gorge du plongeur.
